# EUROPÄISCHE PATENTSCHRIFT

(11) **EP 2 094 145 B1**
(45) Veröffentlichungstag und Bekanntmachung des Hinweises auf die Patenterteilung: **01.01.2014**
(21) Anmeldenummer: 07822022.5
(22) Anmeldetag: 30.10.2007
(51) Int. Cl.: A61L 2/20, B08B 9/00, A47L 15/44, D06F 39/00, A47L 15/42

(54) **VERFAHREN ZUR DESINFEKTION VON LEITUNGSSYSTEMEN EINES WASSER-FÜHRENDEN HAUSHALTSGERÄTS SOWIE DERARTIGES HAUSHALTSGERÄT**
METHOD FOR DISINFECTING CONDUIT SYSTEMS OF A WATER-CONDUCTING HOUSEHOLD APPLIANCE AND SUCH A HOUSEHOLD APPLIANCE
PROCÉDÉ DE DÉSINFECTION DE SYSTÈMES DE CONDUITES D'UN APPAREIL MÉNAGER À DÉBIT D'EAU, ET APPAREIL MÉNAGER DE CE TYPE

(30) Priorität: 09.11.2006 DE 102006052890
(43) Veröffentlichungstag der Anmeldung: 02.09.2009
(73) Patentinhaber: BSH Bosch und Siemens Hausgeräte GmbH, 81739 München (DE)
(72) Erfinder: HEILIGENMANN, Caroline, 71032 Boeblingen (DE); JERG, Helmut, 89537 Giengen (DE)
(86) Internationale Anmeldenummer: PCT/EP2007/061668
(87) Internationale Veröffentlichungsnummer: WO 2008/055811

(56) Entgegenhaltungen:
- EP-A- 1 088 509
- WO-A-2005/039377
- WO-A-2005/063109
- DE-A1- 3 232 057
- DE-A1- 19 513 352
- US-A- 5 960 501

## Beschreibung

Die Erfindung betrifft ein Verfahren zur wenigstens abschnittsweisen Desinfektion von Leitungssystemen eines wasserführenden Haushaltsgeräts sowie eine entsprechend ausgebildete Geschirrspülmaschine und eine Desinfektionseinrichtung.

Bei wasserführenden Haushaltsgeräten, wie z.B. Geschirrspülmaschinen, kann die in der Geschirrspülmaschine umgewälzte Spülflotte in erheblichem Umfang mit organischen Verunreinigungen, wie z.B. Speiseresten etc. belastet sein. Diese Speisereste werden nicht immer vollständig am Ende eines Spülzyklus beim Abpumpen der Spülflotte entfernt. Zurückbleibende Speisereste können unangenehme Gerüche verursachen.

Ein gattungsgemäßes Haushaltsgerät in Form einer Waschmaschine wird in der US 5,960,501 A beschrieben, wobei erfindungsgemäß vorgeschlagen wird, dem Waschwasser in Abhängigkeit der zu waschenden Kleidermenge Ozon zuzugeben.

Eine vergleichbare Lösung schlägt die WO 2005/063109 A1 für Geschirrspülmaschinen vor, wobei Ozon über eine eigene Ozonleitung in den Pumpensumpf oder direkt in den Innenraum der Geschirrspülmaschine eingebracht wird.

Es ist Aufgabe der Erfindung, den Eintrag entsprechender Desinfektionsmittel in die Flüssigkeit zu verbessern.

Diese Aufgabe der Erfindung wird gelöst durch ein Verfahren zur wenigstens abschnittsweisen Desinfektion von einem Leitungssystem eines wasserführenden Haushaltsgerätes, insbesondere einer Geschirrspülmaschine, bei dem in einem Desinfektionsschritt ein gasförmiges Desinfektionsmittel in die im Leitungssystem zirkulierende Flüssigkeit eingespeist wird, wobei erfindungsgemäß vorgesehen ist, dass die Flüssigkeit im Desinfektionsschritt mit einer reduzierten Rate zirkuliert. Dies kann z.B. dadurch erreicht werden, dass eine die Spülflotte umwälzende Umwälzpumpe mit einer reduzierten Drehzahl läuft und so der Austritt von Spülflotte aus Sprüharmen des Leitungssystems einer Geschirrspülmaschine reduziert ist. Zugleich ist aber die Strömungsgeschwindigkeit noch so hoch, dass ausreichend Ozon angesaugt wird, um eine Desinfektion des Leitungssystems zu erreichen. Dabei umfasst im Falle einer Geschirrspülmaschine das Leitungssystem einen in der Bodenwanne der Geschirrspülmaschine angeordneten Pumpentopf, in dem sich die in der Geschirrspülmaschine umgewälzte Spülflotte sammelt. Mittels einer Umwälzpumpe kann die sich im Pumpentopf sammelnde Spülflotte über entsprechende Leitungen des Leitungssystems Sprüharmen zugeführt werden, die zu reinigendes Geschirr und Besteck mit der Spülflotte beaufschlagen. Zur Erwärmung der Spülflotte kann dabei ferner noch ein Durchlauferhitzer vorgesehen sein. Die Spülflotte fließt von dem zu reinigenden Geschirr und Besteck ab und sammelt sich wieder im Pumpentopf. Um eine wenigstens abschnittsweise Desinfektion des Leitungssystems zu erreichen, wird ein gasförmiges Desinfektionsmittel in eine zirkulierende Flüssigkeit eingespeist. Dabei kann es sich bei der zirkulierenden Flüssigkeit um die während eines Reinigungsprogramms umgewälzte Spülflotte handeln, oder der Desinfektionsschritt wird unabhängig von einem Reinigungsprogramm separat durchgeführt, wobei in diesem Fall im Wesentlichen Wasser mit einem gasförmigen Desinfektionsmittel versetzt wird. Entsprechend kann das erfindungsgemäße Verfahren als Einzelschritt standardmäßig im Rahmen von vorgegebenen Reinigungsprogrammen durchgeführt werden oder alternativ manuell bei Bedarf von einer Bedienperson, beispielsweise durch Betätigung eines entsprechenden Betätigungselementes am wasserführenden Haushaltsgerät, ausgelöst werden.

Es können alle geeigneten gasförmigen Desinfektionsmittel, wie z.B. Chlor, Chlordioxid oder Wasserstoffperoxyd Verwendung finden. Vorzugsweise ist jedoch vorgesehen, dass als Desinfektionsmittel Ozon verwendet wird.

Dabei ist in einer bevorzugten Ausführung vorgesehen, dass das Ozon mittels eines Ozongenerators erzeugt wird und daher keine Bevorratung in einem entsprechenden Gasdruckbehälter notwendig ist. Derartige Ozongeneratoren sind bekannt und erzeugen mittels elektrischer Entladungen oder mittels UV-Licht Ozon.

Ferner ist vorzugsweise vorgesehen, dass Ozon unter Unterdruck in die zirkulierende Flüssigkeit eingespeist wird. Hierzu kann beispielsweise eine Venturidüse Verwendung finden. Alternativ kann auch vorgesehen sein, dass Ozon mittels Einpumpen durch eine Pumpe in die zirkulierende Flüssigkeit eingespeist wird.

Grundsätzlich kann die Einspeisung des gasförmigen Desinfektionsmittels an jeder geeigneten Stelle eines Leitungssystems eines wasserführenden Haushaltsgerätes, wie beispielsweise eine Geschirrspülmaschine erfolgen. Vorzugsweise ist jedoch vorgesehen, dass im Desinfektionsschritt Flüssigkeit durch einen Bypass geführt und Ozon im Bypass eingespeist wird.

Ferner gehört zur Erfindung eine Geschirrspülmaschine mit einem Leitungssystem, in dem im Betrieb wenigstens zeitweise Spülflotte zirkuliert. Dabei kann es sich bei dem Leitungssystem einer Geschirrspülmaschine um einen Pumpentopf handeln, indem sich Spülflotte sammelt, die mittels einer Umwälzpumpe durch einen Durchlauferhitzer geführt wird und dann anschließend mittels Sprüharmen auf zu reinigendes Geschirr und Besteck aufgebracht wird.

Die Geschirrspülmaschine ist ferner dadurch gekennzeichnet, dass das Desinfektionsmittel wenigstens ein Gaseinführungsmittel aufweist, das zur Einspeisung von gasförmigen Desinfektionsmitteln in eine im Leitungssystem zirkulierende Flüssigkeit ausgebildet ist. Hierdurch wird es möglich, sowohl während des regulären Betriebs im Rahmen eines Spülprogramms oder alternativ auf Wunsch einer Bedienperson durch manuelle Auslösung einen Desinfektionsvorgang auszulösen.

Es können alle geeigneten gasförmigen Desinfektionsmittel wie z.B. Chlor, Chlordioxid oder Wasserstoffperoxyd Verwendung finden. Vorzugsweise ist jedoch vorgesehen, dass die Desinfektionsmittel wenigstens einen Ozongenerator aufweisen, so dass Ozon bei Bedarf für die Desinfektion erzeugt werden kann und daher eine entsprechende Bevorratung mittels eines gasdichten Behälters entfällt.

Die Gaseinspeisung kann beispielsweise mittels einer Pumpe erfolgen, die das gasförmige Desinfektionsmittel in die zirkulierende Flüssigkeit pumpt. Vorzugsweise ist jedoch vorgesehen, dass das Gaseinführungsmittel zur Einspeisung unter Unterdruck ausgebildet ist. Hierzu ist vorzugsweise vorgesehen, dass das Gaseinführungsmittel eine Venturidüse aufweist, so dass durch die durch die Venturidüse strömende Flüssigkeit ein Unterdruck aufgebaut wird.

In einer bevorzugten Ausführungsform ist dabei vorgesehen, dass das Leitungssystem einen zentralen Sammelbereich, wie beispielsweise einen Pumpentopf aufweist, in dem sich zirkulierende Flüssigkeit sammelt. Ferner ist ein Bypass vorgesehen, der diesen Sammelbereich mit dem Gaseinführungsmittel, wie z.B. einer Venturidüse verbindet.

Dabei weist der Bypass eine Verbindung zu einer Umwälzpumpe auf, um Flüssigkeit im Leitungssystem zirkulieren zu lassen.

Ferner ist vorgesehen, dass im Bypass ein Durchlauferhitzer angeordnet ist. Dabei kann auch alternativ eine Kombination aus einer Umwälzpumpe und einem integrierten Durchlauferhitzer Verwendung finden.

In einer weiteren Ausführungsform ist vorgesehen, dass im Bypass ein Ventil vorgesehen ist. Durch Öffnen des Ventils wird der Bypass frei geschaltet, sodass nun Flüssigkeit durch den Bypass zirkulieren kann, während im normalen Betrieb das Ventil geschlossen ist und den Bypass sperrt.

Ferner ist vorgesehen, dass die Umwälzpumpendrehzahl steuerbar ist. Dies erlaubt einen Betrieb der Umwälzpumpe mit einer reduzierten Drehzahl, wenn Desinfektionsmittel in die zirkulierende Flüssigkeit eingespeist werden. Dabei wird bei einem Betrieb mit der Umwälzpumpe mit reduzierter Drehzahl kein Wasser in den Spülraum einer Geschirrspülmaschine gefördert, es wird jedoch ausreichend Ozon unter Unterdruck in die zirkulierende Flüssigkeit eingespeist.

Im Folgenden wird die Erfindung unter Bezugnahme auf eine Zeichnung erläutert. Es zeigt:

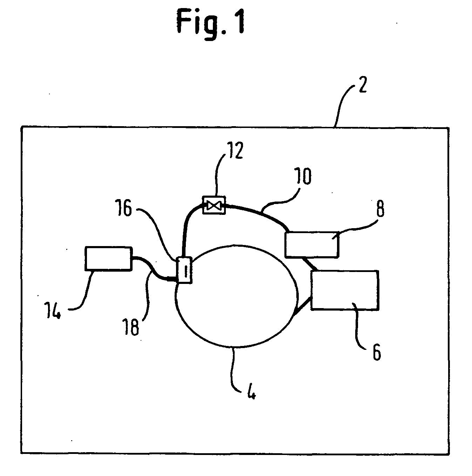

- Fig. 1: eine schematische Ansicht eines Durchschnitts durch eine Bodenwanne einer erfindungsgemäßen Geschirrspülmaschine.

Dargestellt ist eine schematische Darstellung einer Bodenwanne 2 einer Geschirrspülmaschine. Oberhalb der Bodenwanne 2 ist ein Spülbehälter mit einem Spülraum (nicht dargestellt) angeordnet, in dem in Geschirrkörben angeordnetes Geschirr und Besteck angeordnet werden können. In dem Spülraum befinden sich ferner Sprüharme, die zu reinigendes Geschirr und Besteck mit Spülflotte benetzen.

In der Mitte der Bodenwanne 2 ist ein Pumpentopf 4 vorgesehen, der derart angeordnet ist und in Verbindung mit dem Spülraum steht, dass von dem zu reinigenden Geschirr und Besteck ablaufende Spülflotte sich in dem Pumpentopf 4 sammelt.

Ferner sind in der Bodenwanne 2 eine Umwälzpumpe 6, ein Durchlauferhitzer 8, ein Ventil 12, ein Ozongenerator 14 und eine Venturidüse 16 angeordnet. Dabei verbindet ein Bypass 10 den Pumpentopf 4 mit der Umwälzpumpe 6, dem Durchlauferhitzer 8 und der Venturidüse 16 derart, dass Spülflotte aus dem Pumpentopf 4 bei geöffnetem Ventil 12 von der Umwälzpumpe 6 durch den Durchlauferhitzer zur Venturidüse 16 gefördert werden kann. Durch nicht dargestellte weitere Leitungsmittel wird dann Spülflotte von der Venturidüse zu den im Spülraum angeordneten Spülarmen gefördert.

Der Ozongenerator 14 ist mittels einer Leitung 18 mit der Venturidüse 16 verbunden.

Wenn beispielsweise in Rahmen eines Spülprogramms oder durch manuelle Auslösung durch eine Bedienperson der Desinfektionsvorgang des Leitungssystems der Geschirrspülmaschine durchgeführt werden soll, wird seitens einer Steuerung (nicht dargestellt) eine Wasserweiche (nicht dargestellt) geschlossen und das Ventil 12 geöffnet, sodass der Bypass 10 durchgängig ist. Ferner wird nun die Umwälzpumpe 6 mit einer reduzierten Drehzahl betrieben und der Ozongenerator 14 aktiviert. Es fördert die Umwälzpumpe 6 Spülflotte aus dem Pumpentopf 4 über den Durchlauferhitzer 8 und das geöffnete Ventil 12 zu der Venturidüse 16. Dabei ist die Drehzahl der Umwälzpumpe 6 so hoch, dass durch die Strömungsgeschwindigkeit der zirkulierenden Flüssigkeit Ozon durch den Unterdruck in der Venturidüse 18 in ausreichender Menge eingespeist wird. Anschließend strömt die mit Ozon versehene Spülflotte durch ein Rohrsystem (nicht dargestellt) zu den Sprüharmen und bewirkt dort eine Desinfektion durch Oxidation.

Eine Desinfektion des Spülbehälters selbst wird dadurch erreicht, dass Ozon aus der zirkulierenden Spülflotte austritt und so an den Oberflächen des Spülbehälters Schmutz und Bakterien durch Oxidation beseitigt. Nach Abschluss des Desinfektionsschritts geht die Wasserweiche wieder auf ihre offene Position und das Ventil 12 wird geschlossen, so dass die Geschirrspülmaschine wieder für einen normalen Waschzyklus zur Verfügung steht.

### BEZUGSZEICHENLISTE:

- 2: Bodenwanne
- 4: Pumpentopf
- 6: Umwälzpumpe
- 8: Durchlauferhitzer
- 10: Bypass
- 12: Ventil
- 14: Ozongenerator
- 16: Venturidüse
- 18: Zuleitung

## Patentansprüche

1. Verfahren zur wenigstens abschnittsweisen Desinfektion von Leitungssystemen eines wasserführenden Haushaltsgerätes, insbesondere einer Geschirrspülmaschine, bei dem in einem Desinfektionsschritt ein gasförmiges Desinfektionsmittel in eine im Leitungssystem zirkulierende Flüssigkeit eingespeist wird, **dadurch gekennzeichnet, dass** die Flüssigkeit im Desinfektionsschritt mit einer reduzierten Rate zirkuliert.

2. Verfahren nach Anspruch 1, **dadurch gekennzeichnet, dass** als gasförmiges Desinfektionsmittel Ozon verwendet wird.

3. Verfahren nach Anspruch 2, **dadurch gekennzeichnet, dass** das Ozon mittels eines Ozongenerators (14) erzeugt wird.

4. Verfahren nach Anspruch 2 oder 3, **dadurch gekennzeichnet, dass** das Ozon unter Unterdruck in die zirkulierende Flüssigkeit eingespeist wird.

5. Verfahren nach einem der Ansprüche 2 bis 4, **dadurch gekennzeichnet, dass** im Desinfektionsschritt die Flüssigkeit durch einen Bypass (10) geführt und das Ozon im Bypass (10) eingespeist wird.

6. Geschirrspülmaschine mit einem Leitungssystem, in dem im Betrieb wenigstens zeitweise Spülflotte zirkuliert, wobei das Leitungssystem einen in einer Bodenwanne (2) der Geschirrspülmaschine angeordneten Pumpentopf (4) umfasst, in dem sich in der Geschirrspülmaschine umgewälzte Spülflotte sammelt, mit einer Umwälzpumpe (6), mittels derer die sich im Pumpentopf (4) sammelnde Spülflotte über entsprechende Leitungen des Leitungssystems Sprüharmen zugeführt werden kann, die zu reinigendes Geschirr und Besteck mit Spülflotte beaufschlagen, und mit einer Desinfektionseinrichtung, die wenigstens ein Gaseinführungsmittel aufweist, das zur Einspeisung von gasförmigem Desinfektionsmittel in eine im Leitungssystem zirkulierende Flüssigkeit ausgebildet ist, **dadurch gekennzeichnet, dass** die Umwälzpumpendrehzahl steuerbar ist, um einen Betrieb der Umwälzpumpe (6) mit einer reduzierten Drehzahl zu erlauben, wenn Desinfektionsmittel in die zirkulierende Spülflotte eingespeist werden.

7. Geschirrspülmaschine nach Anspruch 6, **dadurch gekennzeichnet, dass** die Desinfektionseinrichtung wenigstens einen Ozongenerator (14) zum Erzeugen von Ozon als Desinfektionsmittel aufweist.

8. Geschirrspülmaschine nach Anspruch 6 oder 7, **dadurch gekennzeichnet, dass** das Gaseinführungsmittel eine Venturidüse (16) ist.

9. Geschirrspülmaschine nach einem der vorhergehenden Ansprüche 6 bis 8, **dadurch gekennzeichnet, dass** das Leitungssystem einen Sammelbereich (4) für die zirkulierende Flüssigkeit aufweist und ein Bypass (10) den Sammelbereich mit den Gaseinführungsmitteln verbindet.

10. Geschirrspülmaschine nach Anspruch 9, **dadurch gekennzeichnet, dass** der Bypass (10) eine Verbindung zu einer Umwälzpumpe (6) aufweist.

11. Geschirrspülmaschine nach Anspruch 9 oder 10, **dadurch gekennzeichnet, dass** der Bypass (10) einen Durchlauferhitzer (8) aufweist.

12. Geschirrspülmaschine nach einem der vorhergehenden Ansprüche 6 bis 11, **dadurch gekennzeichnet, dass** der Bypass (10) ein Ventil (12) aufweist.

## Claims

1. Method for at least sectional disinfection of conduit systems of a water-conducting household appliance, particularly a dishwasher, in which in a disinfecting step a gaseous disinfectant is fed into a liquid circulating in the conduit system, **characterized in that** the liquid circulates at a reduced rate in the disinfection step.

2. Method according to claim 1, **characterized in that** ozone is used as the gaseous disinfectant.

3. Method according to claim 2, **characterized in that** the ozone is generated by means of an ozone generator (14).

4. Method according to claim 2 or 3, **characterized in that** the ozone is fed under negative pressure into the circulating liquid.

5. Method according to one of claims 2 to 4, **characterized in that**, in the disinfecting step, the liquid is conveyed through a bypass (10) and the ozone is fed into the bypass (10).

6. Dishwasher with a conduit system in which washing liquor circulates at least some of the time during operation, wherein the conduit system comprises a pump well (4) disposed in the floor pan (2) of the dishwasher in which washing liquor collects, with a circulation pump (6), by means of which the washing liquor collecting in the pump well (4) can be fed via corresponding conduits of the conduit system to spray arms, which apply washing liquor to crockery and cutlery to be cleaned, and with a disinfection device having at least one gas introduction medium, which is embodied for feeding gaseous disinfectant into a liquid circulating in the conduit system, **characterized in that** the speed of the circulation pump is able to be controlled in order to allow the circulation pump (6) to be operated at a reduced speed when disinfectant is being fed into the circulating washing liquor.

7. Dishwasher according to claim 6, **characterized in that** the disinfecting device has at least one ozone generator (14) for generation of ozone as the disinfectant.

8. Dishwasher according to claim 6 or 7, **characterized in that** the gas introduction medium is a Venturi nozzle (16).

9. Dishwasher according to one of the preceding claims 6 to 8, **characterized in that** the conduit system has a collection area (4) for the circulating liquid and a bypass (10) connects the collection area with the gas introduction medium.

10. Dishwasher according to claim 9, **characterized in that** the bypass (10) has a connection to a circulation pump (6).

11. Dishwasher according to claim 9 or 10, **characterized in that** the bypass (10) has a flow-through heater (8)

12. Dishwasher according to one of the preceding claims 6 to 11, **characterized in that** the bypass (10) has a valve (12).

## Revendications

1. Procédé destiné à la désinfection au moins par sections de systèmes de conduites d'un appareil ménager à circulation d'eau, notamment d'un lave-vaisselle, dans lequel dans une étape de désinfection un moyen de désinfection gazeux est amené dans un liquide circulant dans le système de conduites, **caractérisé en ce que** le liquide circule avec un taux réduit pendant l'étape de désinfection.

2. Procédé selon la revendication 1, **caractérisé en ce que** de l'ozone est utilisé comme produit de désinfection gazeux.

3. Procédé selon la revendication 2, **caractérisé en ce que** l'ozone est produit au moyen d'un générateur d'ozone (14).

4. Procédé selon la revendication 2 ou 3, **caractérisé en ce que** l'ozone est amené dans le liquide en circulation par dépression.

5. Procédé selon l'une quelconque des revendications 2 à 4, **caractérisé en ce que** pendant l'étape de désinfection le liquide est guidé à travers un by-pass (10) et **en ce que** l'ozone est alimenté dans le by-pass (10).

6. Lave-vaisselle comprenant un système de conduites, dans lequel, en fonctionnement, de la flotte de lavage circule au moins temporairement, le système de conduites comprenant un puisard de pompe (4) disposé dans un bac de fond (2) du lave-vaisselle, dans lequel puisard de pompe (4) s'accumule la flotte de lavage en circulation dans le lave-vaisselle, comprenant une pompe de circulation (6) au moyen de laquelle la flotte de lavage s'accumulant dans le puisard de pompe (4) peut être amenée à des bras d'arrosage par l'intermédiaire de conduites correspondantes du système de conduites, lesquels alimentent la vaisselle à nettoyer et les couverts avec de la flotte de nettoyage, et comprenant un dispositif de désinfection qui présente au moins un moyen d'introduction de gaz, qui est réalisé pour l'alimentation de produit de désinfection gazeux dans un liquide circulant dans le système de conduites, **caractérisé en ce que** la vitesse de rotation de la pompe de circulation est commandable afin de permettre un fonctionnement de la pompe de circulation (6) avec une vitesse de rotation réduite lorsque des produits de désinfection sont amenés dans la flotte de nettoyage en circulation.

7. Lave-vaisselle selon la revendication 6, **caractérisé en ce que** le dispositif de désinfection présente au moins un générateur d'ozone (14) destiné à produire de l'ozone comme produit de désinfection.

8. Lave-vaisselle selon la revendication 6 ou 7, **caractérisé en ce que** le moyen d'introduction de gaz est un tube de Venturi (16).

9. Lave-vaisselle selon l'une quelconque des revendications 6 à 8, **caractérisé en ce que** le système de conduites présente une zone d'accumulation (4) pour le liquide en circulation et **en ce qu'**un by-pass (10) relie la zone d'accumulation aux moyens d'introduction de gaz.

10. Lave-vaisselle selon la revendication 9, **caractérisé en ce que** le by-pass (10) présente une liaison vers une pompe de circulation (6).

11. Lave-vaisselle selon la revendication 9 ou 10, **caractérisé en ce que** le by-pass (10) présente un chauffe-eau instantané (8).

12. Lave-vaisselle selon l'une quelconque des revendications 6 à 11, **caractérisé en ce que** le by-pass (10) présente une vanne (12).
